(19) Europäisches Patentamt / European Patent Office / Office européen des brevets

(11) **EP 2 326 328 B1**

(12) **EUROPEAN PATENT SPECIFICATION**

(45) Date of publication and mention of the grant of the patent:
**27.06.2012   Bulletin 2012/26**

(51) Int Cl.:
*A61K 31/502* (2006.01)   *A61K 45/06* (2006.01)
*A61P 9/08* (2006.01)   *A61P 9/10* (2006.01)
*A61P 9/12* (2006.01)   *A61P 11/06* (2006.01)
*A61P 17/00* (2006.01)   *A61P 17/06* (2006.01)
*A61P 25/28* (2006.01)

(21) Application number: **09785769.2**

(22) Date of filing: **05.08.2009**

(86) International application number:
**PCT/HU2009/000073**

(87) International publication number:
**WO 2010/015870 (11.02.2010 Gazette 2010/06)**

(54) **USE OF DIHYDRALAZINE FOR THE TREATMENT OF DISEASES RELATED TO ELEVATED SEMICARBAZIDE SENSITIVE AMINE-OXIDASE (SSAO) ACTIVITY**

VERWENDUNG VON DIHYDRALAZIN ZUR BEHANDLUNG VON ERKRANKUNGEN IM ZUSAMMENHANG MIT ERHÖHTER SEMICARBAZID-SENSITIVER AMINOOXIDASE-(SSAO)-AKTIVITÄT

UTILISATION DE DIHYDRALAZINE POUR TRAITER DES MALADIES ASSOCIÉES À UNE ACTIVITÉ AMINE-OXIDASE SENSIBLE AU SEMI-CARBAZIDE (SSAO) ÉLEVÉE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **06.08.2008   HU 0800498**

(43) Date of publication of application:
**01.06.2011   Bulletin 2011/22**

(73) Proprietor: **Semmelweis Egyetem**
**1085 Budapest (HU)**

(72) Inventors:
• **MÁTYUS, Péter**
  **H-1032 Budapest (HU)**
• **NÉMETH, János**
  **H-1094 Budapest (HU)**
• **MAGYAR, Kálmán**
  **H-1028 Budapest (HU)**
• **SOMOGYI, Anikó**
  **H-1124 Budapest (HU)**
• **DUNKEL, Petra**
  **H-1083 Budapest (HU)**
• **SOMFAI, Gábor Márk**
  **H-1055 Budapest (HU)**
• **BALOGH, Balázs**
  **H-1025 Budapest (HU)**
• **TÚRÓS, György**
  **H-1173 Budapest (HU)**

(74) Representative: **Török, Ferenc**
**Danubia Patent & Law Office LLC**
**POB 198**
**1368 Budapest (HU)**

(56) References cited:
WO-A1-03/055487      WO-A1-2006/002473
WO-A1-2007/054975    WO-A2-02/087508
WO-A2-2006/083779    DE-A1-102006 027 794
US-A1- 2004 005 306   US-A1- 2004 081 642
US-A1- 2008 161 296

• DATABASE WPI Section Ch, Week 200838 Thomson Scientific, London, GB; Class B02, AN 2008-F88706 XP002552311 ZHAI Y.: "Hypotensor compounds for use as medicine for hypertension contains pargyline, reserpine, sulfuric acid dihydralazine, neoflumen and/or chlorobenzene pteridine, dibazol, diazepam, promethazine, rutin and vitamins" & CN 101 091 711 A (NO 2 RETIRET CADRE S LEISURE S [CN]) 26 December 2007 (2007-12-26)

- PASSA P: "The treatment of hypertension in a diabetic patient, when blood pressure is higher or equal to 140/90 mmHg" REVUE DU PRATICIEN - MEDECINE GENERALE 1993 FR, vol. 7, no. 211, 1993, pages 17-20, XP008113996 ISSN: 0989-2737
- SARTORI G: "The treatment of arteriosclerotic macular degeneration in ophthalmology" MEDIZINISCHE MONATSSCHRIFT 1959, vol. 13, no. 8, 1959, pages 496-499, XP008113998 ISSN: 0025-8474
- MATYUS P ET AL: "Semicarnazide-sensitive amine oxidase: Current status and perspectives" CURRENT MEDICINAL CHEMISTRY, BENTHAM SCIENCE PUBLISHERS BV, BE, vol. 11, no. 10, 1 January 2004 (2004-01-01), pages 1285-1298, XP009091626 ISSN: 0929-8673 cited in the application
- SATTLER J ET AL: "Inhibition of human and canine diamine oxidase by drugs used in an intensive care unit: relevance for clinical side effects?" AGENTS AND ACTIONS 1985 CH, vol. 16, no. 3-4, 1985, pages 91-94, XP008114000 ISSN: 0065-4299
- DUNKEL P ET AL: "Semicarbazide-sensitive amine oxidase/vascular adhesion protein 1: Recent developments concerning substrates and inhibitors of a promising therapeutic target" CURRENT MEDICINAL CHEMISTRY, vol. 15, no. 18, August 2008 (2008-08), pages 1827-1839, XP002552310 ISSN: 0929-8673

Remarks:
The file contains technical information submitted after the application was filed and not included in this specification

**Description**

**Field of the invention**

The present invention relates to the use of dihydralazine for the treatment of diseases related to elevated level of SSAO activity and its use for the manufacture of pharmaceutical compositions for the treatment of such diseases.

**Background of the invention**

Semicarbazide sensitive amine oxidase (SSAO) catalyzes the oxidative deamination of primary aliphatic and arylalkylamines, resulting in the formation of the corresponding aldehyde, with the concomitant production of $H_2O_2$ and $NH_3$. The physiological substrates of SSAO are aminoacetone and methylamine. SSAO differ from the other main group of amine oxidases including MAO-A and MAO-B with respect to cofactor, subcellular distribution, biological function, substrates and inhibitors. In the human genome, three SSAO genes have been identified, AOC1 corresponding to diamine oxidase (DAO) (Chassande, O. et al., J. Biol. Chem., 1994, 269, 14484-14489), AOC2 coding for retina-specific SSAO (Imamura, Y. et al., Genomics, 1997, 40, 277-283; Imamura, Y. et al., Genomics, 1998, 51, 293-298; Zhang, Q. et al., Gene, 2003, 318, 45-53), and AOC3 for human placental amine oxidase/VAP-1 (Zhang, X. and McEntire, W.S., Gene, 1996, 179, 279-286; Smith, D.J. et al., J. Exp. Med., 1998, 188, 17-27).
SSAO is involved in the metabolism of biogenic and xenobiotic amines.
The human SSAO enzyme exists as a membrane-bound and as a soluble form in the plasma, its activity displaying a wide tissue distribution. The major sources of the enzyme are the endothelial cells, smooth muscle cells and adipocytes. Sequence identity of SSAO was reported with vascular adhesion protein-1 (VAP-1), which has a role in the adhesion of lymphocytes, therefore in inflammation (Smith, D.J. et al., J. Exp. Med., 1998, 188, 17-27).
Elevated SSAO levels or overexpression of the enzyme have been reported in various pathological conditions (e.g. congestive heart failure (Boomsma, F. et al.. Cardiovasc. Res., 1997, 33, 387-391), end-stage renal disease (Kurkijarvi, R. et al., Eur. J. Immunol., 2001, 31, 2876-2884), inflammatory liver diseases (Kurkijarvi, R. et al., J. Immunol., 1998, 161, 1549-1557), multiple sclerosis (Airas, L. et al., J. Neuroimmunol., 2006, 177, 132-135), psoriasis (Madej, A. et al., J. Eur. Acad. Dermatol. Venerol., 2007, 21, 72-78), Alzheimer's disease (del Mar Hernandez, M. et al., Neurosci. Lett., 2005, 384, 183-187; Ferrer, I. et al., 2002, 321, 21-24) and myopathies (Olive, M. et al., Muscle Nerve, 2004, 29, 261-266)).
Elevated levels of SSAO activity have been observed in type I and type II diabetes, markedly in the presence of diabetic complications (Boomsma, F. et al., Biochim. Biophys. Acta, 2003, 1647, 48-54; Boomsma, F. et al., Clin. Sci., 1995, 88, 675-679; Garpenstrand, H. et al., Diabetic. Med., 1999, 16, 514-521; Mészáros, Z. et al., Metab. Clin. Exp., 1999, 48, 113-117; Boomsma, F. et al., Diabetologia, 1999, 42, 233-237; Salmi, M. et al., Am. J. Pathol., 2002, 161, 2255-2262). Elevated SSAO activity has been reported in diabetic retinopathy, supporting the hypothesis that SSAO may be involved in the pathogenesis of this diabetic complication.
Thus, pharmacological inhibition of the SSAO enzyme may exert beneficial effects in various diseases.
As a consequence of the elevated activity of the enzyme in diabetes, there is an increased formation of the cito- and/or angiotoxic enzyme products, that may contribute to the pathogenesis of diabetic complications. Since there is a high expression of SSAO in the endothel and the plasma, the SSAO-related citotoxic effects might be more significant in the highly vascularised tissues, such as for example in the eyes (Ekblom, J., Pharmacol. Res., 1998, 37, 87-92). The citotoxic enzyme products in case of the physiological substrates are formaldehyde, methylglioxal, $NH_3$ and $H_2O_2$, that may increase the deleterious effects of oxidative stress, moreover may lead to the formation of protein cross-links and advanced-glycation end-products (AGEs). The said processes have an important role also in the pathogenesis of diabetic complications. Thus, inhibitors of SSAO activity may be useful for the treatment of diabetic complications, particularly diabetic retinopathy and diabetic macular edema.
Diabetic retinopathy and diabetic macular edema are severe complications of diabetes even in an epidemiological aspect. Diabetic retinopathy is the second leading cause of acquired blindness in adults. Currently, the primary treatment of diabetic retinopathy and macular edema is retinal laser photocoagulation therapy. However, this therapy is relatively time consuming besides leading to a beneficial outcome only in a moderate number of cases, mostly helping to reduce the progression of the disease. A routine treatment is pars plana vitrectomy, i.e. surgical clearing of the vitreous body. The surgical technique however is rather elaborate, costly and has certain risks. On the other hand, the currently available pharmacological therapies are not effective enough, they can lead only to a transient amelioration of the condition. Therefore, there is a great demand for novel pharmacological agents, that are more effective than the currently available therapies (Ciulla, A.T. et al., Diabetes Care, 2003, 26, 2653-2664).
Of the potential pharmacological therapies of retinopathy, clinical trials have been conducted with AGE inhibitors and antioxidants (Soro-Paavonen, A., Curr. Med. Chem., 2006, 13, 1777-1788). Retinal leukostasis, capillary occlusion and on the other hand, chronic, low-grade subclinical inflammation were suggested to have central and causal role in the formation of the signature vascular lesions of diabetic retinopathy (Joussen, A.M., FASEB J., 2001, 18, 1450).

Inhibition of the SSAO activity leads to a decrease in the formation of AGEs and in oxidative stress. Moreover, a specific VAP-1 inhibitor was reported to have beneficial effects in an uveitis model (endotoxin-induced uveitis) (Noda, K., FASEB J., 2008, 22, 1094-1103). Thiazole VAP-1 inhibitors were disclosed as useful for the treatment of macular edema (WO 2006/028269, WO 2006/011631, WO 2004/067521). In an animal model of diabetes, the treatment with the inhibitor exhibited a beneficial effect on the increased vascular permeability in the eyes (WO 2004/067521). The strong SSAO inhibitor aminoguanidine has beneficial effect on diabetic retinopathy in animal model (Ellis, E.N., et al., Metabolism, 1991, 40, 1016), its effect might be related to its SSAO inhibition.

Considering the above detailed aspects, it is reasonable to suggest that application of pharmaceutical compositions of SSAO/VAP-1 inhibitors could be a novel, effective approach for the treatment of inflammatory eye diseases. Currently there is not any drug available in therapy exploiting this approach.

In SSAO enzyme catalysis the carbonyl group containing topaquinone cofactor has a central role, analysis of its structure may serve as a sound basis for the design of inhibitor compounds. The name of SSAO derives from its sensitivity to inhibition by semicarbazide. Besides semicarbazide, the well-known inhibitor of SSAO, also other classes of compounds were described as inhibitors, such as several hydrazine derivatives. Among the drugs already used in therapy containing hydrazino group, several were reported to have SSAO inhibitory effect, such as hydralazine, carbidopa and benserazide (Mátyus, P. et al., Curr. Med. Chem., 2004, 11, 1285-1298). None of these compounds has been used however for the treatment of diseases related to elevated SSAO activity, said diseases including diabetic complications, such an application of these drugs was not even suggested previously.

An underlying reason of that could be, that the primary effect of these drugs that serve as the basis of their recent use in therapy (e.g. antihypertensive effect), may be a serious adverse effect in course of their use for the treatment of diseases related to elevated SSAO activity, such as retinopathy.

The growing interest in hydrazino derivatives however is well illustrated by the recent disclosure of several SSAO inhibitor hydrazino derivatives, e.g. some hydrazinoalcohol derivatives (WO 2005/080319, WO 2002/002090); hydrazinoindane derivatives (WO 2003/006003); and phenylallylhydrazine derivatives (Wang, E.Y. et al., J. Med. Chem., 2006, 49, 2166-2173, WO 2006/094201). According to the patent publications, these compounds could be useful for the treatment of acute and chronic inflammations, diseases related to carbohydrate metabolism, diseases related to adipocyte differentiation or dysfunction, diseases related to smooth muscle dysfunctions and vascular diseases.

WO 03/0055487 discloses the use of dihydralazine in the treatment of diseases associated with damage mediated by an $\alpha,\beta$-unsaturated aldehyde, including diabetic complications.

Dihydralazine has been used in the treatment of age-related macular degeneration (Sartori, Medizische Monatsschrift 1959, vol 13, no 8, 496-499).

**Disclosure of the invention**

Dihydralazine has long been used in human therapy as an antihypertensive agent. Despite its structural analogy with hydralazine (dihydralazine may be regarded as a substituted hydralazine), no data have been published previously on the effect of dihydralazine on SSAO. In spite of the structural resemblance of dihydralazine to hydralazine, the SSAO inhibitory activity of dihydralazine is far from being evident, due to the observations that even slight structural modifications in hydrazine derivatives may significantly influence the type and magnitude of SSAO inhibitory activity (Lizcano, J. M. et al., Biochem. Pharmacol., 1996, 52, 187-195). The SSAO inhibition by dihydralazine was confirmed by our experiments described in the examples. Therapeutic application of dihydralazine as an SSAO inhibitor may be more advantageous than that of hydralazine, as a consequence of the presence of one more hydrazino group in dihydralazine. It is well known that the hydrazino group of hydralazine and one of the hydrazino groups of dihydralazine undergo metabolic inactivation (Schneider, T. et al., Pharmazie, 1988, 43, 33-36, ibidem. 704-706), the other, metabolically intact hydrazino group of dihydralazine may therefore be available for SSAO inhibition that may result in particularly preferable pharmacokinetic and phamacodynamic properties of dihydralazine as SSAO inhibitor.

The present invention relates to the use of dihydralazine in the treatment of a disease related to elevated levels of SSAO activity, namely, in the treatment of diabetic macular edema.

It should be noted, that according to our animal experiments, dihydralazine is able to exhibit significant SSAO inhibition in such a low dose, that does not cause appreciable (i.e. appreciable to the animal or human treated) blood pressure lowering effect. Thus, in a preferred embodiment of the invention dihydralazine is used for the inhibition of SSAO activity in such a dose, which does not cause appreciable lowering in blood pressure in the animal or human treated under the normal levels as defined in the claims. However, in patients with hypertension, lowering of blood pressure, i.e. antihypertensive effect could be beneficial, therefore, in case of such patients, even higher doses (depending on the degree of hypertension) than the ones described below as preferred embodiments of the invention can be used.

In one embodiment, the invention relates to the use of dihydralazine in therapy per se or with one or more additional compounds in combination. In another embodiment, dihydralazine can be used *per se* or in combination with one or more additional compounds for the manufacture of a pharmaceutical composition.

**Detailed description of the invention**

The pharmaceutical compositions prepared on the basis of the present invention can be administered by any means that achieve their intended purpose. Routes of administration of the present invention can be, for example, oral, intravenous, intramuscular, subconjunctival, parabulbar, retrobulbar, subtenon, intracameral, intravitreal and other injections, sublingual, transdermal, ocular, eye drops, eye gels or ointments. Compositions of the present invention can be solid, semisolid or liquid.

Potential liquid compositions of the present invention include but are not limited to solutions, tinctures, syrups, emulsions and suspensions.

The pharmaceutical compositions of the present invention can contain one or more non-toxic, pharmaceutically acceptable excipients (e.g. agents used for formulation, carriers, surface acting agents, colours, sweeteners, solvents, suspending agents, coatings etc.). Preferred are controlled-release compositions and compositions providing an organ specific targeted delivery of the active ingredient.

Pharmaceutical compositions of the present invention can be prepared by admixing the active ingredient dihydralazine or its salt (preferred are for example sulphate, hydrochloride salts) with one or more excipients and by formulation to a pharmaceutical composition. Pharmaceutical compositions can be prepared by methods and contain excipients which are well known in the art. A generally recognized compendium of such methods and ingredients is Remington's Pharmaceutical Sciences.

In one embodiment, dihydralazine is administered in a dose of 0.005-1.5 mg/bodyweight kg. In a preferred embodiment, dihydralazine is administered in a dose of 0.2-1 mg/bodyweight kg, in a more preferred embodiment, in a dose of 0.4-0.8 mg/bodyweight kg, if the preferred embodiment of administering the active ingredient without causing appreciable lowering of blood pressure is intended to be carried out. These dosage intervals are related to administration to humans and can be used for the administration to animals just with the appropriate interpolation.

Pharmaceutical compositions of the invention may contain single doses. The exact dosage administered will be dependent upon a variety of factors and can be chosen by the individual physician in view of the appropriate parameters (weight of the recipient, age, stage and degree of the disease etc).

As demonstrated by Example 3, in adults of average weight (approx. 70 kg), administration of 14.5 mg dihydralazine sulphate two times daily was found to be preferable (no appreciable lowering of blood pressure was observed).

The effect of dihydralazine was studied in Micromedex/Drugdex evaluations. In the treatment of hypertension, dihydralazine is indicated as supplemental therapy as a second or third drug. Dihydralazine therapy is initiated with lower doses (eg, 12.5 to 25 milligrams twice daily), with gradual dose increases based on clinical response and tolerability to total daily doses of 25 to 100 milligrams in 2 or 3 divided doses (Strocchi E. et al., Int. J. Clin. Pharmacol. Ther. Toxicol., 1983, 21, 519-523; Komajda M. et al., Am. J. Nephrol., 1986, 6(suppl 2), 106-112; Thibonnier M. et al., Br. J. Clin. Pharmacol., 1980, 9, 561-567; Salmela P.I. et al., Ann. Clin. Res., 1981, 13, 433-438; Sassano P. et al., Am. J. Med., 1987, 83, 227-235). Higher doses (150 milligrams/day) have been used in some studies (Kirch W. & Axthelm T., J. Cardiovasc. Pharmacol., 1982, 4, 562-566).

In one embodiment, the invention relates to the use of dihydralazine in therapy per se or with one or more additional active ingredients in combination. In another embodiment, dihydralazine can be used *per se* or in combination with one or more additional active ingredients for the manufacture of a pharmaceutical composition. Preferred additional active ingredients of the invention are agents inhibiting SSAO activity and/or anti-inflammatory agents and/or VEGF inhibitors and/or VEGF small interfering RNA agents and/or tyrosine kinase inhibitors and/or protein kinase C inhibitors and/or angiotensin converting enzyme (ACE) inhibitors and/or PPAR-γ inhibitors and/or acetylsalicylic acid and/or statins (statin type compounds, abbreviated: statins), and/or compounds used for the treatment of diabetes and/or its pathological consequences.

Preferred additional active ingredients can be: VEGF inhibitors, as for example pegaptanib (Macugen), ranibizumab (Lucentis), bevacizumab (Avastin), vatalanib; VEGF Trap; VEGF small interfering RNA agents, as for example bevasiranib, Sima-027; PPAR-γ inhibitors, as for example glitazones; tyrosine kinase inhibitors, as for example ALN-VEG01, AG-013958; steroid or non-steroid anti-inflammatory agents.

The invention is illustrated by the following non-restrictive experimental examples.

**Example 1**

Study of the effect of dihydralazine on human serum SSAO activity

In the experiment human serum was used in the following way: blood samples were collected without anticoagulant, then stored at 4°C for 15 minutes and centrifuged (4°C, 2500 g/10 min). The supernatant was stored at -80°C until the measurement of the enzyme activity.

A radiochemical procedure (Yu P.H., Zuo D.M., Biochem. Pharmacol., 1992, 43, 307-312) was adapted for determining SSAO activities in the human serum. It is based on the determination of the quantity of $^{14}$C-benzaldehyde formed by the enzyme reaction of $^{14}$C-benzylamine.

The preparations containing the serum and the dihydralazine enzyme inhibitor were pre-incubated at room temperature for 20 min. The enzyme reaction was started with $^{14}$C-benzylamine substrate (- 200000 dpm/tube). During the experiments 200 $\mu$l was the final volume of the incubation media, the composition of which is shown in Table 1. The samples obtained accordingly were incubated at 37°C for 40 min, then the reaction was stopped by adding 200 $\mu$l 2M citric acid solution. In case of the 'blank' sample, citric acid solution was added before adding the substrate, therefore, in this case no enzyme reaction took place. The samples were extracted with 1000 $\mu$l 1:1 toluene - ethyl acetate mixture, by vortex method, then centrifuged (2500 g / 5 min). The enzyme activity was determined by liquid scintillation counting (600 $\mu$l supernatant + 5ml Aquasafe), using the formula below:

$$\frac{[dpm\ (sample) - dpm\ 0']\ x\ 1.6\ x\ 10\ x\ 1.5}{measured\ substrate\ activity\ dpm\ x\ mg\ protein}$$

standing for: 1.6: extraction volume; 10: molar activity; 1.5: correction factor for the incubation time; dpm: disintegrations per minute (unit of radioactivity)

The inhibition was calculated in the % inhibition compared to the control ('control' sample not containing the inhibitor compound) after correction with the background ('blank' sample).

The method used for the determination of the protein quantity of the samples is based on comparison of the 1000× dilution of the plasma to the standard calibration curve of serum albumin (Bradford M.M., Anal. Biochem. 72, 248, 1976). The % inhibition can be determined also of the dpm values of the control and the sample.

**Table 1.**

| Composition of the incubation media | | | |
|---|---|---|---|
| Solutions | 'blank' (0') | 'control' | sample |
| 0.1 M phosphate buffer, pH=7.4 | 100 $\mu$l | 100 $\mu$l | 100 $\mu$l |
| 1 mM clorgyline.HCl | 20 $\mu$l | 20 $\mu$l | 20 $\mu$l |
| inhibitor (dihydralazine) | - | - | 20 $\mu$l |
| serum | 40 $\mu$l | 40 $\mu$l | 40 $\mu$l |
| 0.5 mM $^{14}$C-benzylamine (BZA) | 20 $\mu$l | 20 $\mu$l | 20 $\mu$l |
| distilled water | 20 $\mu$l | 20 $\mu$l | - |
| | | | |
| [2 M citric acid (to stop the enzyme reaction) | 200 $\mu$l | 200 $\mu$l | 200 $\mu$l |

According to the assay, the $IC_{20}$ value of dihydralazine on human serum SSAO is 0.5 $\mu$M.

**Example 2**

Effect of dihydralazine on the SSAO activity *in vivo* in rats

Rats were treated sc with 0.1 or 0.01 M dihydralazine solution (0.1 ml/100 g) once daily for 1-5 days. On the appropriate day, 2 hours after the treatment the rats were decapitated and their blood was collected without anticoagulant in plastic centrifuge tubes. Blood samples were centrifuged at a low frequency (800 rpm/min) and the supernatant was used for the determination of SSAO activity applying $^{14}$C-benzylamine substrate.

SSAO inhibition expressed in pmol/mg protein/hour units and in % values (mean $\pm$ S.D.) in the two treated groups is shown in table 2 and 3.

The blood pressure of the animals was determined in an invasive way. In treated animals, compared to the control group, the treatment did not cause lowering of the blood pressure.

**Table 2**

| Effect of dihydralazine on SSAO activity *in vivo* (1.) | | | | | | | |
|---|---|---|---|---|---|---|---|
| **Serum SSAO activity in case of 10 μmol/kg dose (1.9 mg/kg)** | | | | | | | |
| Group | Specific activity (pmol/mg/h) | Mean | SD | p (t-test) | Inhibition (%) | Mean | SD |
| Control | 3.52<br>3.08<br>3.52 | 3.373 | 0.252 | | -4<br>9<br>-4 | 0 | 7 |
| 10 μmol/kg 24 h | 2.11<br>1.19<br>1.44 | 1.580 | 0.477 | 0.010 | 37<br>65<br>57 | 53 | 14 |
| 10 μmol/kg 48 h | 1.52<br>1.66<br>1.61 | 1.595 | 0.067 | 0.004 | 55<br>51<br>52 | 53 | 2 |
| 10 μmol/kg 72 h | 1.25<br>1.06<br>1.43 | 1.244 | 0.187 | 0.000 | 63<br>69<br>58 | 63 | 6 |
| 10 μmol/kg 96 h | 0.97<br>1.94<br>1.12 | 1.344 | 0.523 | 0.010 | 71<br>42<br>67 | 60 | 16 |
| 10 μmol/kg 120 h | 0.78<br>2.90<br>0.59 | 1.425 | 1.284 | 0.114 | 77<br>14<br>82 | 58 | 38 |

**Table 3**

| Effect of dihydralazine on SSAO activity *in vivo* (2.) | | | | | | | |
|---|---|---|---|---|---|---|---|
| **Serum SSAO activity in case of 100 μmol/kg dose (19 mg/kg)** | | | | | | | |
| Group | Specific activity (pmol/mg/h) | Mean | SD | p (t-test) | Inhibition (%) | Mean | SD |
| Control | 3.52<br>3.08<br>3.52 | 3.373 | 0.252 | | -4<br>9<br>-4 | 0 | 7 |
| 100 μmol/kg 24 h | 1.13<br>1.05<br>1.11 | 1.094 | 0.041 | 0.003 | 67<br>69<br>67 | 68 | 1 |
| 100 μmol/kg 48 h | 1.31<br>1.33<br>1.80 | 1.481 | 0.279 | 0.001 | 61<br>60<br>47 | 56 | 8 |
| 100 μmol/kg 72 h | 1.11<br>1.26<br>1.15 | 1.177 | 0.079 | 0.002 | 67<br>63<br>66 | 65 | 2 |
| 100 μmol/kg 96 h | 0.55<br>1.12<br>0.91 | 0.859 | 0.288 | 0.000 | 84<br>67<br>73 | 75 | 9 |
| 100 μmol/kg 120 h | 1.29<br>0.34<br>0.56 | 0.730 | 0.495 | 0.004 | 62<br>90<br>83 | 78 | 15 |

**Example 3**

Study of the effect of dihydralazine on the macular edema of type 2 diabetic patients and on the SSAO activity

The effect of orally administered low dose dihydralazine on macular edema and serum SSAO activity was studied in type 2 diabetic patients in an early stage of macular edema formation.

After giving their informed consent, patients participating in the study were treated for 56 days taking ½ tablet twice daily of a pharmaceutical composition containing 28.9 mg dihydralazine sulphate (i.e. the ½ tablet contained 14.5 mg dihydralazine sulphate).

To measure the efficacy and monitor the safety of the treatment, the following examinations were carried out before starting the treatment and at the 1., 7., 14., 28. and 56. day of the treatment:

1. ocular examinations: visual acuity (ETDRS chart), slit-lamp examination, measurement of intraocular pressure with applanation tonometry, fundus biomicroscopy with pupil dilation, digital fundus photography. The grade of macular edema was determined via measuring retinal thickness with optical coherency tomography (OCT).
2. internal medicine examinations: anamnesis, physical examinations, blood pressure, pulse, urine examination.
3. laboratory examinations: blood glucose, HbA1c, liver function, kidney function, microalbuminuria, metabolic panel of the blood , lipids, CRP.

Serum SSAO activity was determined from blood samples collected before starting the treatment, and at the 14., 28. and 56. day of the treatment. The blood samples were centrifuged and the supernatant was stored at - 80°C until the measurement of the enzyme activity. SSAO activity was determined applying the method described in the example above.

The table below (Table 4) summarizes the data of 2 treated patients. In the presented cases, significant decrease of the macular edema was observed in the fovea, the most important anatomical location of the retina responsible for central sharp vision, with a parallel decrease of serum SSAO activity. As illustrated by the data, the applied dose did not cause significant change of blood pressure.

In the table the following abbreviations were used:

BMI: body mass index - weight (kg) / height (m$^2$)
RRsyst: blood pressure (systolic) (Hgmm)
RRdiast: blood pressure (diastolic) (Hgmm)
OCT CST (central subfield thickness): the average thickness of the central 1-mm area of the macula
OCT CPT (center point thickness): the thickness of the center point of the retina; normally that is the thinnest point of the macula

**Table 4.**

| Data of the patients' treated | | |
|---|---|---|
| | 1. patient | 2. patient |
| Gender | male | male |
| Age | 74 | 64 |
| BMI (kg/m$^2$) | 25.2 | 29.6 |
| Blood pressure (Hgmm) | | |
| before treatment | | |
| RRsyst | 153 | 153 |
| RRdiast | 63 | 76 |
| pulse | 60 | 79 |
| 14. day | | |
| RRsyst | 154 | 150 |
| RRdiast | 70 | 65 |

(continued)

| Data of the patients' treated | | |
| --- | --- | --- |
| | 1. patient | 2. patient |
| pulse | 69 | 89 |
| 28. day | | |
| RRsyst | 152 | 158 |
| RRdiast | 68 | 74 |
| pulse | 69 | 93 |
| 56. day | | |
| RRsyst | 161 | 126 |
| RRdiast | 67 | 64 |
| pulse | 60 | 77 |
| OCT CST | | |
| before treatment | 298 | 302 |
| 14. day | 266 | 323 |
| 28. day | 273 | 286 |
| 56. day | 265 | 252 |
| OCT CPT | | |
| before treatment | 272 | 257 |
| 14. day | 215 | 319 |
| 28. day | 219 | 239 |
| 56. day | 209 | 201 |
| SSAO activity (pmol/mg protein/hour) | | |
| before treatment | 93.02 | 167.56 |
| 14. day | 62.42 | 127.96 |
| 28. day | 85.73 | 186.36 |
| 56. day | 82.41 | 105.1 |

**Claims**

1. Dihydralazine for use in the treatment of diabetic macular edema.

2. Dihydralazine for use according to claim 1, wherein dihydralazine is to be administered in a 0.005-1.5 mg/weight kg, preferably in a 0.2-1 mg weight kg, more preferably in a 0.4-0.8 mg/weight kg dose.

3. Dihydralazine for use according to claim 1 or 2, wherein dihydralazine is to be administered in combination with one or more additional active ingredients, preferably in combination with other agents inhibiting SSAO activity and/or anti-inflammatory agents and/or VEGF inhibitors and/or VEGF small interfering RNA agents and/or tyrosine kinase inhibitors and/or protein kinase C inhibitors and/or angiotensin converting enzyme (ACE) inhibitors and/or PPAR-$\gamma$ inhibitors and/or acetylsalicylic acid and/or statin type compounds and/or compounds used for the treatment of diabetes and/or its pathological consequences.

**Patentansprüche**

1.  Dihydralazin zur Verwendung in der Behandlung von diabetischem makularem Ödem.

2.  Dihydralazin zur Verwendung nach Anspruch 1, worin Dihydralazin in einer Dosis von 0,005-1,5 mg/Gewichtkg, vorzugsweise in einer Dosis von 0,2-1 mg/Gewichtkg, optimalerweise in einer Dosis von 0,4-0,8 mg/Gewichtkg dosiert werden soll.

3.  Dihydralazin zur Verwendung nach Anspruch 1 oder 2, worin Dihydralazin in Kombination mit einer oder mehreren aktiven Komponenten, vorzugsweise in Kombination mit die SSAO-Aktivität inhibierenden anderen Stoffen und/ oder antiinflammatorischen Stoffen und/oder VEGF-Inhibitoren und/oder mit VEGF interferierenden kleinen RNS Stoffen und/oder Tyrosinkinase-Inhibitoren und/oder Proteinkinase C Inhibitoren und/oder Inhibitoren des Angiotensin-konvertierendem Enzyms (ACE) und/oder PPAR-$\gamma$-Inhibitoren und/oder Acetylsalicylsäure und/oder Verbindungen Statin-Typs und/oder zur Behandlung von Zuckerkrankheit verwendeten Verbindungen und/oder ihren pathologischen Konsequenzen dosiert werden soll.

**Revendications**

1.  Dihydralizine à utiliser dans le traitement de l'oedème maculaire diabétique.

2.  Dihydralizine à utiliser selon la Revendication 1, **caractérisée en ce que** la dihydralazine est à administrer en une dose de 0,005-1,5 mg/kg pondéral, de préférence de 0,2-1 mg/kg pondéral, de façon optimale en une dose de 0,4-0,8 mg/kg pondéral.

3.  Dihydralizine à utiliser selon la Revendication 1 ou 2, **caractérisée en ce que** la dihydralazine est à administrer en combinaison avec un ou plusieurs ingrédients actifs, de préférence en combinaison avec d'autres agents inhibant l'activité SSAO et/ou avec des agents anti-inflammatoires et/ou avec des inhibiteurs du VEGF et/ou avec des agents de petits ARN interférents avec VEGF et/ou avec des inhibiteurs de tyrosine kinase et/ou avec des inhibiteurs d'enzyme de conversion de l'angiotensine (ACE) et/ou avec des inhibiteurs de PPAR-$\gamma$ et/ou avec de l'acide acétylsalicylique et/ou avec des composés de type statine et/ou avec des composés utilisés pour le traitement du diabète et/ou des conséquences pathologiques de celui-ci.

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006028269 A
- WO 2006011631 A
- WO 2004067521 A
- WO 2005080319 A
- WO 2002002090 A
- WO 2003006003 A
- WO 2006094201 A
- WO 030055487 A

**Non-patent literature cited in the description**

- **CHASSANDE, O. et al.** *J. Biol. Chem.,* 1994, vol. 269, 14484-14489
- **IMAMURA, Y. et al.** *Genomics,* 1997, vol. 40, 277-283
- **IMAMURA, Y. et al.** *Genomics,* 1998, vol. 51, 293-298
- **ZHANG, Q. et al.** *Gene,* 2003, vol. 318, 45-53
- **ZHANG, X. ; MCENTIRE, W.S.** *Gene,* 1996, vol. 179, 279-286
- **SMITH, D.J. et al.** *J. Exp. Med.,* 1998, vol. 188, 17-27
- **BOOMSMA, F. et al.** *Cardiovasc. Res.,* 1997, vol. 33, 387-391
- **KURKIJARVI, R. et al.** *Eur. J. Immunol.,* 2001, vol. 31, 2876-2884
- **KURKIJARVI, R. et al.** *J. Immunol.,* 1998, vol. 161, 1549-1557
- **AIRAS, L. et al.** *J. Neuroimmunol.,* 2006, vol. 177, 132-135
- **MADEJ, A. et al.** *J. Eur. Acad. Dermatol. Venerol.,* 2007, vol. 21, 72-78
- **DEL MAR HERNANDEZ, M. et al.** *Neurosci. Lett.,* 2005, vol. 384, 183-187
- **OLIVE, M. et al.** *Muscle Nerve,* 2004, vol. 29, 261-266
- **BOOMSMA, F. et al.** *Biochim. Biophys. Acta,* 2003, vol. 1647, 48-54
- **BOOMSMA, F. et al.** *Clin. Sci.,* 1995, vol. 88, 675-679
- **GARPENSTRAND, H. et al.** *Diabetic. Med.,* 1999, vol. 16, 514-521
- **MÉSZÁROS, Z. et al.** *Metab. Clin. Exp.,* 1999, vol. 48, 113-117
- **BOOMSMA, F. et al.** *Diabetologia,* 1999, vol. 42, 233-237
- **SALMI, M. et al.** *Am. J. Pathol.,* 2002, vol. 161, 2255-2262
- **EKBLOM, J.** *Pharmacol. Res.,* 1998, vol. 37, 87-92
- **CIULLA, A.T. et al.** *Diabetes Care,* 2003, vol. 26, 2653-2664
- **SORO-PAAVONEN, A.** *Curr. Med. Chem.,* 2006, vol. 13, 1777-1788
- **JOUSSEN, A.M.** *FASEB J.,* 2001, vol. 18, 1450
- **NODA, K.** *FASEB J.,* 2008, vol. 22, 1094-1103
- **ELLIS, E.N. et al.** *Metabolism,* 1991, vol. 40, 1016
- **MÁTYUS, P. et al.** *Curr. Med. Chem.,* 2004, vol. 11, 1285-1298
- **WANG, E.Y. et al.** *J. Med. Chem.,* 2006, vol. 49, 2166-2173
- **SARTORI.** *Medizische Monatsschrift,* 1959, vol. 13 (8), 496-499
- **LIZCANO, J. M. et al.** *Biochem. Pharmacol.,* 1996, vol. 52, 187-195
- **SCHNEIDER, T. et al.** *Pharmazie,* 1988, vol. 43, 33-36
- *PHARMAZIE,* 704-706
- **STROCCHI E. et al.** *Int. J. Clin. Pharmacol. Ther. Toxicol.,* 1983, vol. 21, 519-523
- **KOMAJDA M. et al.** *Am. J. Nephrol.,* 1986, vol. 6 (2), 106-112
- **THIBONNIER M. et al.** *Br. J. Clin. Pharmacol.,* 1980, vol. 9, 561-567
- **SALMELA P.I. et al.** *Ann. Clin. Res.,* 1981, vol. 13, 433-438
- **SASSANO P. et al.** *Am. J. Med.,* 1987, vol. 83, 227-235
- **KIRCH W. ; AXTHELM T.** *J. Cardiovasc. Pharmacol.,* 1982, vol. 4, 562-566
- **YU P.H. ; ZUO D.M.** *Biochem. Pharmacol.,* 1992, vol. 43, 307-312
- **BRADFORD M.M.** *Anal. Biochem.,* 1976, vol. 72, 248